(19) Europäisches Patentamt
European Patent Office
Office européen des brevets

(11) Numéro de publication: 0 337 837 B1

(12) # FASCICULE DE BREVET EUROPEEN

(45) Date de publication du fascicule du brevet: 29.08.90

(51) Int. Cl.[5]: C07C 319/14

(21) Numéro de dépôt: 89400842.4

(22) Date de dépôt: 24.03.89

(54) Procédé de préparation de disulfures et polysulfures organiques.

(30) Priorité: 14.04.88 FR 8804962
14.09.88 FR 8811968

(43) Date de publication de la demande: 18.10.89 Bulletin 89/42

(45) Mention de la délivrance du brevet: 29.08.90 Bulletin 90/35

(84) Etats contractants désignés: AT BE CH DE ES FR GB GR IT LI LU NL SE

(56) Documents cités:
FR-A- 1 381 265

CHEMICAL ABSTRACTS, vol. 107, no. 5, août 1987, abrégé no. 39367c, Columbus, Ohio, US

(73) Titulaire: SOCIETE NATIONALE ELF AQUITAINE (PRODUCTION), Tour Elf 2, Place de la Coupole La Défense 6, F-92400 Courbevoie(FR)

(72) Inventeur: Arretz, Emmanuel, 2, Rue de Cagnes, F-64000 Pau(FR)

(74) Mandataire: Leboulenger, Jean et al, ATOCHEM Département Propriété Industrielle, F-92091 Paris la Défense 10 Cédex 42(FR)

EP 0 337 837 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la préparation de disulfures et polysulfures organiques par action du soufre sur un mercaptan ou sur un polysulfure de rang inférieur en soufre, en présence d'un catalyseur basique.

L'oxydation de mercaptans par le soufre est une importante voie d'accès aux disulfures organiques. Cette sulfuration directe avec élimination d'hydrogène sulfuré selon la réaction:

$$2\ RSH + S \rightarrow RSSR + H_2S$$

est généralement mise en oeuvre en phase liquide et catalysée par des agents basiques organiques ou inorganiques, en particulier des bases alcalines ou des amines. Quand elle est effectuée avec du soufre solide en présence d'une amine (brevet US 2 237 625), cette réaction est essentiellement réalisée en discontinu et s'accompagne de la formation de polysulfures supérieurs.

Pour éviter l'emploi de soufre solide, il a été proposé dans le brevet FR 1 358 398 de mettre le mercaptan directement en contact avec une solution de soufre dans un solvant, ce dernier étant de préférence un disulfure organique et principalement le disulfure que l'on désire produire. Dans ce procédé où la solution de soufre dans le disulfure est préparée à l'avance dans un bac spécial, l'amine sert non seulement à catalyser la réaction d'oxydation, mais aussi à accroître la solubilité du soufre dans le disulfure et donc à obtenir des solutions très concentrées en soufre.

Une amélioration importante à cette voie de synthèse des disulfures organiques a fait l'objet du brevet FR 2 130 985. Elle consiste à introduire dans le réacteur muni d'un agitateur le soufre à l'état liquide au-dessus de la phase réactionnelle liquide contenant en solution le catalyseur basique, par exemple une amine telle que la triéthylamine. Cette façon de procéder permet d'obtenir des disulfures organiques exempts de tout autre polysulfure et de réaliser un procédé continu simplifié.

Les polysulfures organiques $RS_nR$ ($n > 2$), notamment aliphatiques, cycloaliphatiques ou aryliques, et tout particulièrement les dialkylpolysulfures, sont des produits d'intérêt industriel pour diverses applications, en particulier comme additifs extrême-pression dans les huiles de coupe.

Leur préparation par réaction entre le soufre élémentaire et un mercaptan est connue. Cette réaction:

$$2\ RSH + (n-1)\ S \rightarrow RS_nR + H_2S$$

nécessite la présence d'un catalyseur basique qui peut être une amine, une alcanolamine, une base inorganique, un mercaptide ou un alcoolate, ou encore un catalyseur constitué par la combinaison d'un mercaptan avec un oxyde d'alcène et une base alcaline. De tels procédés ont été décrits par exemple dans les brevets US 2 237 625, 2 237 627, 3 022 351 et 3 392 201 ainsi que dans les brevets FR 1 381 265, GB 1 162 334, DE 2 938 156 et FR 2 607 496.

L'emploi d'oxyde de magnésium comme catalyseur a été récemment revendiqué dans le brevet US 4 564 709. Etant solide et insoluble dans les milieux réactionnels liquides obtenus par réaction du soufre élémentaire avec des mercaptans ou avec des polysulfures organiques, un tel catalyseur présente l'avantage de ne pas contaminer les polysulfures produits dans ces réactions. Cependant, l'oxyde de magnésium utilisé sous forme de poudre nécessite une filtration très efficace des produits de réaction, ce qui rend très délicate l'exploitation d'un tel procédé pour une fabrication en continu de polysulfures organiques.

L'obtention de polysulfures $RS_nR$ peut être aussi réalisée par l'action du soufre élémentaire sur des polysulfures organiques $RS_{n'}R$ de rang inférieur en soufre ($2 \leqq n' < n$), par exemple des di-, tri- ou tétrasulfures pour former des polysulfures supérieurs. Cette possibilité est signalée par exemple dans le brevet FR 1 381 265, en présence d'amines comme catalyseurs.

Il a maintenant été trouvé qu'aussi bien dans la préparation des disulfures $RS_2R$ par action du soufre sur un mercaptan que dans celle des polysulfures $RS_nR$ par action du soufre sur un mercaptan ou un polysulfure de rang inférieur en soufre, on peut utiliser comme catalyseurs basiques des résines échangeuses d'anions. Ces résines solides se présentent généralement sous la forme de grains ou de billes facilement séparables. Etant insolubles dans les milieux réactionnels liquides, ces catalyseurs peuvent être utilisés de manière permanente dans les réacteurs, sans appoint continu, et présentent l'avantage de ne pas polluer les effluents sortant du réacteur.

L'invention a donc pour objet un procédé de préparation de disulfures et polysulfures organiques par action du soufre sur un mercaptan en milieu liquide ou sur un polysulfure organique de rang inférieur en soufre, caractérisé en ce qu'on utilise comme catalyseur une résine échangeuse d'anions.

La présente invention s'applique en premier lieu à la préparation des disulfures et polysulfures de dialkyle contenant de 2 à 40 atomes de carbone tels que les disulfures et polysulfures de diméthyle, diéthyle, dipropyle, dibutyle, dipentyle, dihexyle, diheptyle, dioctyle, didécyle ou didodécyle. Elle s'applique également à la préparation de disulfures et polysulfures cycloalkyliques (par exemple, disulfure ou polysulfure de dicyclohexyle), arylalkyliques (par exemple, disulfure ou polysulfure de dibenzyle) ou aromatiques (par exemple, disulfure ou polysulfure de diphényle). Le procédé selon l'invention permet aussi de

fabriquer des disulfures et polysulfures fonctionnalisés à partir de mercaptans dont le radical hydrocarboné R porte un ou plusieurs groupes fonctionnels tels que, par exemple, les atomes d'halogène et les groupes OH, OR′, SR′, $NH_2$, NHR′, NR′R″, CN, CHO, COR′, COOH ou COOR′, R′ et R″ désignant des radicaux aliphatiques, cycloaliphatiques, aromatiques ou alkylaromatiques.

Les catalyseurs solides utilisés selon l'invention sont des polymères ou copolymères organiques à fonction basique, bien connus dans l'art comme échangeurs d'anions. Comme tels, on peut employer plus particulièrement des résines à base de polystyrène réticulées notamment avec du divinylbenzène, des résines acryliques ou phénylacryliques, des résines acryliques réticulées avec du divinylbenzène, ou des résines du type phénol-formaldéhyde. Ces résines portent des groupes fonctionnels amine tertiaire ou ammonium quaternaire fixés par différentes techniques connues en soi, généralement après formation des polymères ou copolymères. On peut également mentionner les résines époxy-amines obtenues par réaction directe entre l'ammoniac et l'épichlorhydrine, les polyvinylpyridines obtenues par polymérisation de la vinvl-4 pyridine, et les résines obtenues par aminolyse d'acrylates par des polyamines. Des résines échangeuses d'anions se trouvent dans le commerce sous différentes dénominations telles que, par exemple, Amberlite, Amberlyst, Dowex, Duolite, Lewatit, Reillex,....

Les résines échangeuses d'anions portant des groupes fonctionnels ammonium quaternaire sont livrées par les fabricants de résines sous la forme ionique chlorure pour garantir un maximum de stabilité au stockage et au transport. Leur basicité étant totalement neutralisée sous cette forme, ces résines doivent, préalablement à leur emploi comme catalyseurs, être traitées par une base telle que l'hydroxyde de sodium pour recouvrer leurs propriétés basiques sous la forme de groupes fonctionnels d'hydroxydes d'ammonium quaternaire. Dans la pratique les résines de départ sous forme chlorure sont traitées par une solution appropriée d'hydroxyde de sodium de façon à éliminer tous les ions chlorure, puis elles sont lavées à l'eau jusqu'à élimination complète de l'hydroxyde de sodium.

L'efficacité des résines échangeuses d'anions, qu'elles soient de type amine ou bien de type ammonium quaternaire (sous la forme $OH^-$) se trouve généralement améliorée lorsqu'on les utilise sèches. Leur activité catalytique se manifeste généralement à partir d'une quantité minimale de 0,1 % en poids par rapport au soufre introduit dans le milieu réactionnel. Dans la plupart des cas, la quantité maximale utile est de l'ordre de 50 % en poids par rapport au soufre utilisé, mais une quantité plus importante peut s'avérer utile dans certains cas, notamment pour la préparation de disulfures et polysulfures de dialkyles secondaires ou tertiaires. La quantité préférée est cependant généralement comprise entre 5 et 40% en poids.

Le procédé selon l'invention peut être mis en oeuvre dans un réacteur, muni d'un agitateur, où le catalyseur est en suspension dans le milieu réactionnel liquide. Dans ce cas, toutes les techniques d'introduction du soufre sous différentes formes peuvent être employées : soufre à l'état solide, soufre à l'état liquide, solution de soufre dans un solvant sélectif, solution de soufre dans un disulfure ou polysulfure (de préférence celui à fabriquer).

Le procédé selon l'invention peut également être mis en oeuvre au moyen d'un réacteur tubulaire dans lequel le catalyseur est disposé en lit fixe, en lit mobile ou en lit expansé. Dans ce mode de réalisation, le soufre est introduit soit à l'état liquide, soit en solution sous les formes diverses précédemment énumérées (solvant sélectif, disulfure ou polysulfure).

La réaction proprement dite peut avoir lieu dans un large domaine de températures suivant le mercaptan à transformer et le type de résine basique employée. Elle est en général effectuée à une température comprise entre - 10°C et la température limite de stabilité thermique de la résine utilisée. Même pour les résines basiques faibles les plus stables, il est préférable de ne pas dépasser 100°C, notamment dans le cas d'une utilisation prolongée de la résine.

La réaction peut être conduite à pression atmosphérique. Cependant, dans le cas d'un mercaptan volatil comme le méthylmercaptan, il est préférable que le soufre soit en contact avec le mercaptan liquide ; l'application d'une pression suffisante pour éviter l'entraînement du mercaptan avec l'hydrogène sulfuré formé peut alors être favorable à la réaction. Par contre, dans le cas de mercaptans peu volatils, à haut point d'ébullition, on peut envisager l'introduction d'un gaz inerte (par exemple l'azote) à travers le mélange réactionnel ou maintenir dans le réacteur une dépression adéquate de façon à faciliter l'élimination de l'hydrogène sulfuré dès sa formation et favoriser ainsi au maximum la réaction.

Le rapport molaire mercaptan/soufre à mettre en oeuvre peut aller de 0,3 à 6 et est choisi en fonction de la nature du mercaptan utilisé et du produit à fabriquer (disulfure ou polysulfure).

Pour la synthèse d'un disulfure suivant la réaction :

$$2R\text{-}SH + S \rightarrow R\text{-}SS\text{-}R + H_2S$$

le rapport molaire mercaptan/soufre doit être au moins égal à 2. Cependant, pour obtenir une excellente sélectivité en disulfure et minimiser ainsi la formation concomitante de polysulfures, il convient généralement d'opérer avec un excès de mercaptan par rapport à la stoechiométrie ; le rapport molaire mercaptan/soufre est de préférence compris entre 3 et 5.

La réaction de formation des polysulfures ($n \geq 3$) s'écrivant globalement :

$$2R\text{-}SH + (n\text{-}1)S \rightarrow R\text{-}S_n\text{-}R + H_2S$$

le rang en soufre (n) du polysulfure obtenu varie en fonction inverse du rapport molaire mercaptan/soufre mis en oeuvre. D'autre part, dans le cas des mercaptans primaires ou secondaires, il est avantageux de travailler avec un rapport molaire faible (au plus égal à 1, et de préférence, inférieur à 1) de façon à éviter la rétrogradation des polysulfures en disulfure. Par contre, dans le cas de mercaptans tertiaires comme, par exemple, le tertiobutylmercaptan ou le tertiooctylmercaptan, le rapport molaire mercaptan/soufre peut aller jusqu'à 3 sans inconvénient.

Dans le cas où l'on part d'un polysulfure organique (par exemple di-, tri-, tétrasulfure...), la quantité de soufre à utiliser est déterminée en fonction de ce polysulfure de départ et du polysulfure de rang supérieur en soufre à synthétiser. Le rapport molaire entre les deux réactifs: polysulfure organique de départ et soufre peut varier de 0,2 à 1.

Les exemples suivants illustrent l'invention sans la limiter.

## EXEMPLE 1: Diméthyldisulfure

Le réacteur utilisé est muni d'une agitation centrale et d'une double enveloppe extérieure. Il est équipé pour travailler soit à pression atmosphérique soit sous pression. Le volume utile de réaction est de l'ordre de 300 ml.

### Premier essai

On introduit dans le réacteur 94 g de diméthyldisulfure et 32 g de soufre solide. On ajoute ensuite, sous agitation, 5 g de résine anionique à groupements fonctionnels amine tertiaire (Amberlyst A 21 produite par Röhm and Haas), préalablement séchée. Après dissolution du soufre, on injecte, par l'intermédiaire d'un tube plongeant, dans le milieu liquide agité, du méthylmercaptan gazeux avec un débit de 24 1/h pendant 2 heures (soit environ 2 moles au total), la température de réaction étant maintenue à 30°C. Après séparation de la résine, le liquide réactionnel est traité par un courant d'azote pour éliminer le méthylmercaptan non consommé et l'hydrogène sulfuré formé qui est resté dissous. Par pesée et analyse du mélange liquide récupéré on détermine la production de diméthyldisulfure, à savoir 31 g, soit un rendement de 32 % par rapport au soufre introduit.

### Deuxième essai

Dans le même réacteur refroidi à -10°C, on condense 192 g de méthylmercaptan introduit sous forme gazeuse. On introduit successivement dans le méthylmercaptan liquide 32 g de soufre solide et 5 g de résine Amberlyst A21. On observe rapidement un dégagement gazeux constitué d'hydrogène sulfuré et de méthylmercaptan entraîné. On maintient sous agitation le mélange à la température de -10°C et, au bout d'une heure, on sépare la résine du mélange réactionnel. Après élimination par un courant d'azote du méthylmercaptan restant et de l'hydrogène sulfuré dissous, on obtient 85 g d'un liquide dont l'analyse montre qu'il se compose de 95 % de diméthyldisulfure, le reste étant constitué de polysulfures organiques. Le rendement en diméthyldisulfure est de 85,9 % par rapport au soufre introduit.

### Troisième essai

Dans le même réacteur que précédemment, on introduit 32 g de soufre solide et 5 g de résine anionique Amberlyst A 21, puis le réacteur est pressurisé avec de l'azote jusqu'à une pression de 3 bars absolus. On ajoute ensuite 192 g de méthylmercaptan liquide, et on maintient le milieu réactionnel à une température de 30°C, sous agitation et à une pression de 3 bars absolus au moyen d'une vanne pneumatique de détente dont la sortie à pression atmosphérique permet l'élimination des gaz produits dans la réaction. Au bout d'une heure, on récupère le brut de réaction séparé de la résine et on élimine le méthylmercaptan et l'hydrogène sulfuré dissous. On obtient 90 g d'un liquide dont l'analyse montre qu'il se compose de 95,2 % de diméthyldisulfure, le reste étant constitué de polysulfures. Le rendement en diméthyldisulfure est de 91,1 % par rapport au soufre introduit.

### Quatrième essai

Le réacteur contenant au départ 5 g de résine Amberlyst A 21 est chargé sous une pression de 3 bars absolus d'azote avec 192 g de méthylmercaptan liquide. Au moyen d'un système d'injection approprié, on introduit ensuite sous agitation 32 g de soufre liquide sur une période de 20 minutes, le réacteur étant maintenu à une température de 40°C et à une pression de 3 bars absolus. Les effluents gazeux sont éliminés en continu par la vanne de détente à pression atmosphérique. Au bout de 10 minutes on récupère le produit de réaction séparé de la résine et on le traite par un courant d'azote pour éliminer le méthylmercaptan et l'hydrogène sulfuré dissous. On obtient 92 g de liquide dont l'analyse montre qu'il se compose

de 97 % de diméthyldisulfure, le reste étant constitué de polysulfures. Le rendement en diméthyldisulfure est de 94,9 % par rapport au soufre introduit.

EXEMPLE 2: Diéthyldisulfure

Dans le même réacteur qu'à l'exemple 1, on introduit 186 g d'éthylmercaptan, 24 g de soufre et 4 g de résine Amberlyst A 21 anhydre. Le mélange est ensuite agité pendant une heure, à la température de 30°C. Après séparation de la résine, on obtient un liquide dont l'analyse montre qu'il contient 87 g de diéthyldisulfure, ce qui représente un rendement en diéthyldisulfure de 95 % par rapport au soufre introduit.

Cette opération a été reproduite avec d'autres résines échangeuses d'anions dans des conditions identiques (mêmes quantités d'éthylmercaptan, de soufre et de résine ; température : 30°C).

Les produits IRA-400 et A-26 sont des résines à groupements fonctionnels ammonium quaternaire, et les produits IRA-94 S et IRA-93 SP des résines à groupements fonctionnels amine tertiaire.

Les résultats obtenus sont présentés dans le tableau suivant :

| Résine | Temps de réaction (heure) | Rendement (%) en diéthyldisulfure |
|---|---|---|
| A-21 | 1 | 95 |
| IRA-400 | 1 | 40 |
| | 3 | 90 |
| A-26 | 1 | 97 |
| IRA-94 S | 1 | 98 |
| IRA-93 SP | 1 | 98,5 |

EXEMPLE 3: Di(n-propyl)disulfure

On opère comme à l'exemple 2, mais en remplaçant l'éthylmercaptan par 228 g de n-propylmercaptan. Le rendement en di(n-propyl)disulfure est de 9% par rapport au soufre introduit.

EXEMPLE 4: Di(n-dodécyl)disulfure

Le même réacteur qu'à l'exemple 1 est chargé de 202 g de n-dodécylmercaptan, de 8 g de soufre et de 3 g de résine Amberlyst A 21. A température ambiante, la formation de disulfure est faible. Par contre, en augmentant la température, on observe un dégagement important d'hydrogène sulfuré et après avoir maintenu le milieu réactionnel à 75°C pendant une heure, on récupère un liquide dont l'analyse montre qu'il contient essentiellement du disulfure de dodécyle et le n-dodécylmercaptan mis en excès. Le rendement en di(n-dodécyl)disulfure est de 98 % par rapport au soufre introduit.

EXEMPLE 5: Diphényl disulfure

On introduit dans le même réacteur que précédemment 220 g de thiophénol, 16 g de soufre et 4 g de résine Amberlyst A 21. Le mélange est maintenu sous agitation à température ambiante. A la fin du dégagement d'hydrogène sulfuré, on récupère le liquide dont l'analyse montre qu'il est constitué uniquement de diphényldisulfure et de thiophénol mis en excès. Le rendement en diphényldisulfure est de 99 % par rapport au soufre introduit.

EXEMPLE 6: Di-isopropyl disulfure

Le même réacteur que dans l'exemple 1 est chargé de 190 g d'isopropylmercaptan, de 20 g de soufre et de 3 g de résine Amberlyst A 21. A température ambiante, la formation de disulfure représente environ 40 % de la proportion théorique. Le réacteur est alors légèrement pressuré par de l'azote (0,4 bar absolu) et le milieu réactionnel est chauffé et maintenu agité à une température de 60°C pendant une heure. Le brut de réaction est ensuite analysé. Le di-isopropyldisulfure a été obtenu avec un rendement de 93%.

EXEMPLE 7: Bis(hydroxy-2 éthyl) disulfure

Dans le même réacteur qu'à l'exemple 1, on introduit 195 g de mercaptoéthanol, 20 g de soufre solide et 7,6 g de résine Amberlyst A-21 anhydre. Le mélange est ensuite agité pendant 1 heure à température ambiante, puis chauffé à 85°C pendant une demi-heure. Après séparation de la résine, on obtient un liquide

dont l'analyse montre qu'il contient essentiellement du bis(hydroxyéthyl)disulfure et le mercaptoéthanol mis en excès. Le rendement en ce disulfure est de 97,5 % par rapport au soufre introduit.

EXEMPLE 8: Dithio-3,3' dipropionate de méthyle

Dans le même réacteur qu'à l'exemple 1, on charge 240 g de mercapto-3 propionate de méthyle, 16 g de soufre solide et 7 g de résine Amberlyst A-21 anhydre. On agite le mélange pendant une heure à la température ambiante, puis on chauffe à 85°C pendant une heure et demie.

On obtient ainsi le disulfure $(CH_3O\text{-}CO\text{-}CH_2CH_2)_2\ S_2$ avec un rendement de 96 % par rapport au soufre introduit.

EXEMPLE 9: Diméthylpolysulfure à partir du méthylmercaptan

Dans un réacteur ayant un volume utile de réaction de l'ordre de 600 ml, muni d'une agitation centrale et d'une double enveloppe extérieure, et équipé pour travailler soit à pression atmosphérique soit sous pression, on introduit 64 g de soufre solide et 6 g de résine Amberlyst A21 sèche, puis le réacteur est pressurisé avec de l'azote jusqu'à une pression de 3 bars absolus. On ajoute ensuite 48 g de méthylmercaptan liquide et on maintient le mélange réactionnel à 30°C, sous agitation et à une pression de 3 bars absolus au moyen d'une vanne pneumatique de détente dont la sortie à pression atmosphérique permet d'éliminer les gaz produits par la réaction. Au bout d'une heure, on sépare la résine du brut réactionnel qu'on traite par un courant d'azote pour éliminer le méthylmercaptan non consommé et l'hydrogène sulfuré dissous.

On obtient ainsi 85 g d'un liquide dont l'analyse montre qu'il se compose d'un mélange de polysulfures ayant la répartition pondérale suivante :
diméthyldisulfure: 1,5%
diméthyltrisulfure: 27,7%
diméthyltétrasulfure: 30,9%
diméthylpentasulfure: 24,3%
polysulfures supérieurs: 15,6%

EXEMPLE 10: Diméthylpolysulfure à partir de diméthyldisulfure

Dans le même réacteur qu'à l'exemple 9, on introduit 64 g de soufre solide, 8 g de résine Amberlyst A21 et 94 g de diméthyldisulfure, puis on maintient le mélange à pression atmosphérique, sous agitation et à 60°C pendant 2 heures.

Après séparation de la résine et élimination des gaz dissous, on récupère 152 g d'un liquide dont l'analyse indique la composition pondérale suivante :
diméthyldisulfure: 2,1%
diméthyltrisulfure: 47,2%
diméthyltétrasulfure: 31,4%
diméthylpentasulfure: 18,1%
polysulfures supérieurs: 1,2%

EXEMPLE 11: Ditertiobutylpolysulfure à partir du tertiobutylmercaptan

On a effectué une série de trois essais en utilisant 52 g de soufre, 10 g de résine Amberlyst A21 sèche et une quantité variable (440 g, 293 g ou 73,3 g) de tertiobutylmercaptan (TBM). Chaque essai a été réalisé à pression atmosphérique, sous agitation et à 60°C, sur une durée de 3 heures.

Les résultats obtenus sont présentés dans le tableau suivant :

| Rapport molaire TBM/S | COMPOSITION EN DITERTIOBUTYLPOLYSULFURES $C_4H_9-S_n-C_4H_9$ (% poids) | | | | |
|---|---|---|---|---|---|
| | n = 2 | n = 3 | n = 4 | n = 5 | n > 5 |
| 3/1 | 4,1 | 59,3 | 30,7 | 5,9 | - |
| 2/1 | 1,1 | 27,8 | 61,5 | 9,2 | 0,4 |
| 1/2 | - | 10,8 | 72,1 | 16,1 | 1 |

Une série de trois essais effectués dans les mêmes conditions, mais en remplaçant la résine Amberlyst A21 par la résine Amberlite IRA 93 SP (résine anionique à groupements fonctionnels amine tertiaire) a donné des résultats pratiquement équivalents.

EXEMPLE 12: Ditertiooctylpolysulfure à partir du tertiooctylmercaptan

En utilisant 32 g de soufre, 10 g de résine Amberlyst A21 sèche et une quantité variable (439 g, 292,6 g ou 73,2 g) de tertiooctylmercaptan (TOM), on a effectué trois essais réalisés chacun à pression atmosphérique, sous agitation à 80°C, pendant 3 heures.

Le tableau suivant présente les résultats obtenus :

| Rapport molaire TOM/S | COMPOSITION EN DITERTIOOCTYLPOLYSULFURES $C_8H_{17}-S_n-C_8H_{17}$ (% molaires déterminés par RMN) | | | | |
|---|---|---|---|---|---|
| | n = 3 | n = 4 | n = 5 | n = 6 | n > 6 |
| 3/1 | 44 | 56 | - | - | - |
| 2/1 | 34 | 66 | - | - | - |
| 1/2 | 7 | 48 | 20 | 11 | 14 |

EXEMPLE 13: Ditertiononylpolysulfure à partir du tertiononylmercaptan

Dans le même réacteur qu'à l'exemple 9, on introduit 32 g de soufre solide, 5 g de résine Amberlyst A21 sèche et 80 g de tertiononylmercaptan obtenu à partir d'un trimère du propylène. Le mélange est ensuite maintenu sous agitation à pression atmosphérique et à 80°C pendant 3 heures.

Après séparation de la résine et dégazage du liquide par un courant d'azote, on obtient un ditertiononylpolysulfure dont la teneur en soufre est de 37,9 %, ce qui correspond à un rendement de 98,2 % par rapport à la théorie.

EXEMPLE 14: Ditertiododécylpolysulfure à partir du tertiododécylmercaptan

On opère comme à l'exemple 13, mais en remplaçant le tertiononylmercaptan par 101 g de tertiododécylmercaptan obtenu à partir d'un tétramère du propylène.

La teneur en soufre du ditertiododécylpolysulfure ainsi obtenu est de 31,3 %, ce qui correspond à un rendement de 97,4 % par rapport à la théorie.

EXEMPLE 15: Bis(hydroxy-2 éthyl) polysulfure

On introduit dans le réacteur 46,8 g de mercaptoéthanol, 19,2 g de soufre solide et 2 g de résine Amberlyst A-21 sèche, puis on agite le mélange pendant une heure à température ambiante et une heure à 85°C.

Après séparation de la résine et dégazage, on obtient un liquide dont l'analyse indique la composition molaire suivante :
bis(hydroxy-2 éthyl) disulfure: 12%
bis(hydroxy-2 éthyl) trisulfure: 51%
bis(hydroxy-2 éthyl) polysulfures supérieurs: 37%

EXEMPLE 16: Polythio-3,3' dipropionate de méthyle

On opère comme à l'exemple 15, mais en remplaçant le mercaptoéthanol par 72 g de mercapto-3 propionate de méthyle et en portant à une heure et demie la durée de chauffage à 85°C.

L'analyse du liquide obtenu montre qu'il est constitué d'un mélange des polysulfures de formule :

$$(CH_3O\overset{\overset{O}{\|}}{C}-CH_2CH_2)_2S_n$$

dans les proportions molaires suivantes :

| n | % |
|---|---|
| 2 | 15 |
| 3 | 47 |
| ≥4 | 38 |

**Revendications**

1. Procédé de préparation de disulfures et polysulfures organiques par action du soufre sur un mercaptan ou sur un polysulfure de rang inférieur en soufre, en milieu liquide et en présence d'un catalyseur basique, caractérisé en ce qu'on utilise comme catalyseur une résine échangeuse d'anions.

2. Procédé selon la revendication 1, dans lequel la quantité de résine mise en oeuvre est comprise entre 0,1 et 50 % en poids par rapport au soufre introduit, de préférence entre 5 et 40 %.

3. Procédé selon la revendication 1 ou 2, dans lequel on opère à une température comprise entre -10°C et la température limite de stabilité thermique de la résine utilisée, de préférence au-dessous de 100°C.

4. Procédé selon l'une des revendications 1 à 3, dans lequel la résine échangeuse d'anions est une résine à groupes fonctionnels amine tertiaire ou ammonium quaternaire.

5. Procédé selon l'une des revendications 1 à 4, dans lequel le mercaptan est un alkyl-, cycloalkyl-, aryl- ou aralkyl-mercaptan.

6. Procédé selon la revendication 5, dans lequel le radical hydrocarboné du mercaptan porte un ou plusieurs groupes fonctionnels.

7. Procédé selon l'une des revendications 1 à 6, dans lequel le rapport molaire mercaptan/soufre est compris entre 0,3 et 6.

8. Procédé selon l'une des revendications 1 à 6 pour la préparation de disulfures, caractérisé en ce que le rapport molaire mercaptan/soufre est compris entre 2 et 6, de préférence entre 3 et 5.

9. Procédé selon l'une des revendications 1 à 6 pour la préparation de polysulfures dérivés de mercaptans primaires ou secondaires, caractérisé en ce que le rapport molaire mercaptan/soufre est compris entre 0,3 et 1.

10. Procédé selon l'une des revendications 1 à 6 pour la préparation de polysulfures dérivés de mercaptans tertiaires, caractérisé en ce que le rapport molaire mercaptan/soufre est compris entre 0,3 et 3.

11. Procédé selon l'une des revendications 1 à 4 pour la préparation de polysulfures $RS_nR$ à partir d'un polysulfure $RS_{n'}R$ ($2 \leq n' < n$), caractérisé en ce que le rapport molaire du polysulfure $RS_{n'}R$ au soufre introduit est compris entre 0,2 et 1.

## Patentansprüche

1. Verfahren zur Herstellung von organischen Disulfiden und Polysulfiden durch Reaktion von Schwefel mit einem Mercaptan oder einem weniger Schwefel enthaltenden Polysulfid in flüssigem Milieu und in Gegenwart eines basischen Katalysators, dadurch gekennzeichnet, daß man als Katalysator ein Anionenaustauscherharz verwendet.

2. Verfahren nach Anspruch 1, in dem die eingesetzte Harzmenge zwischen 0,1 und 50 Gew.-% im Verhältnis zum eingebrachten Schwefel, vorzugsweise zwischen 5 und 40%, beträgt.

3. Verfahren nach Anspruch 1 oder 2, in dem man bei einer Temperatur zwischen –10°C und einer durch die thermische Stabilität des verwendeten Harzes begrenzten Temperatur, vorzugsweise unter 100°C, arbeitet.

4. Verfahren nach einem der Ansprüche 1 bis 3, in dem das Anionenaustauscherharz ein Harz mit funktionellen tertiären Amin- oder quartären Ammoniumgruppen ist.

5. Verfahren nach einem der Ansprüche 1 bis 4, in dem das Mercaptan ein Alkyl-, Cycloalkyl-, Aryl- oder Aralkylmercaptan ist.

6. Verfahren nach Anspruch 5, in dem sich am Kohlenwasserstoffrest des Mercaptans eine oder mehrere funktionelle Gruppen befinden.

7. Verfahren nach einem der Ansprüche 1 bis 6, in dem das Molverhältnis zwischen Mercaptan und Schwefel zwischen 0,3 und 6 liegt.

8. Verfahren nach einem der Ansprüche 1 bis 6 zur Herstellung von Disulfiden, dadurch gekennzeichnet, daß das Molverhältnis zwischen Mercaptan und Schwefel zwischen 2 und 6, vorzugsweise zwischen 3 und 5 liegt.

9. Verfahren nach einem der Ansprüche 1 bis 6 zur Herstellung von von primären oder sekundären Mercaptanen abgeleiteten Polysulfiden, dadurch gekennzeichnet, daß das Molverhältnis zwischen Mercaptan und Schwefel zwischen 0,3 und 1 liegt.

10. Verfahren nach einem der Ansprüche 1 bis 6 zur Herstellung von von tertiären Mercaptanen abgeleiteten Polysulfiden, dadurch gekennzeichnet, daß das Molverhältnis zwischen Mercaptan und Schwefel zwischen 0,3 und 3 liegt.

11. Verfahren nach einem der Ansprüche 1 bis 4 zur Herstellung von Polysulfiden $RS_nR$, ausgehend von einem Polysulfid $RS_{n'}R$ ($2 \leq n' < n$), dadurch gekennzeichnet, daß das Molverhältnis zwischen Polysulfid $RS_{n'}R$ und eingebrachtem Schwefel zwischen 0,2 und 1 liegt.

## Claims

1. Process for the preparation of organic disulphides and polysulphides by the action of sulphur on a mercaptan or on a polysulphide of a lower order in respect of sulphur, in a liquid medium and in the presence of a basic catalyst, characterized in that an anion exchange resin is used as catalyst.

2. Process according to claim 1, in which the amount of resin used is between 0.1 and 50% by weight relative to the sulphur introduced, and preferably between 5 and 40%.

3. Process according to claim 1 or 2, in which the reaction is carried out at a temperature of between –10°C and the limiting temperature at which the resin used is still stable to heat, preferably below 100°C.

4. Process according to one of claims 1 to 3, in which the anion exchange resin is a resin containing tertiary amine or quaternary ammonium functional groups.

5. Process according to one of claims 1 to 4, in which the mercaptan is an alkyl, cycloalkyl, aryl or aralkylmercaptan.

6. Process according to claim 5, in which the hydrocarbon radical of the mercaptan carries one or more functional groups.

7. Process according to one of claims 1 to 6, in which the mercaptan/sulphur molar ratio is between 0.3 and 6.

8. Process according to one of claims 1 to 6 for the preparation of disulphides, characterized in that the mercaptan/sulphur molar ratio is between 2 and 6 and preferably between 3 and 5.

9. Process according to one of claims 1 to 6 for the preparation of polysulphides derived from primary or secondary mercaptans, characterized in that the mercaptan/sulphur molar ratio is between 0.3 and 1.

10. Process according to one of claims 1 to 6 for the preparation of polysulphides derived from tertiary mercaptans, characterized in that the mercaptan/sulphur molar ratio is between 0.3 and 3.

11. Process according to one of claims 1 to 4 for the preparation of polysulphides $RS_nR$ from a polysulphide $RS_{n'}R$ ($2 \leq n' < n$), characterized in that the molar ratio of polysulphide $RS_{n'}R$ to sulphur introduced is between 0.2 and 1.